# EUROPEAN PATENT APPLICATION

(11) **EP 4 678 108 A1**
(43) Date of publication of application: **14.01.2026**
(21) Application number: 25188506.7
(22) Date of filing: 09.07.2025
(51) Int. Cl.: A61B 6/02, A61B 6/50

(54) **IMAGE PROCESSING APPARATUS, IMAGE PROCESSING METHOD, AND IMAGE PROCESSING PROGRAM**

(30) Priority: 12.07.2024 JP 2024112900
(71) Applicant: FUJIFILM Corporation, Tokyo 106-8620 (JP)
(72) Inventor: FUKUDA, Waturu, Kanagawa, 258-8538 (JP)
(74) Representative: Dehns Germany Partnerschaft mbB

(57) **Abstract**

An image processing apparatus (16) includes a CPU. The CPU acquires a two-dimensional image captured by irradiating a breast (U) with radiation; acquires a position of a radiation source that emits the radiation in a case in which the two-dimensional image is captured; acquires a series of a plurality of projection images or a plurality of tomographic images obtained by performing tomosynthesis imaging of the breast (U); calculates a virtual projection position, which is a position at which the breast (U) is virtually projected during the tomosynthesis imaging, from the position of the radiation source in a case in which the two-dimensional image is captured; and generates a composite two-dimensional image from the plurality of projection images or the plurality of tomographic images based on the virtual projection position.

## Description

### BACKGROUND OF THE INVENTION

### 1. Field of the Invention

The present disclosure relates to an image processing apparatus, an image processing method, and an image processing program.

### 2. Description of the Related Art

A technology of generating a radiation image corresponding to a normal two-dimensional image obtained by normal imaging by combining a series of a plurality of projection images obtained by tomosynthesis imaging performed by irradiating a breast with radiation or a plurality of tomographic images generated from the series of projection images is known. For example, JP7203705B discloses an image processing apparatus that generates a plurality of composite two-dimensional images having different generation methods from a plurality of tomographic images acquired by tomosynthesis imaging.

### SUMMARY OF THE INVENTION

In a case of interpreting a radiation image, particularly, a radiation image obtained by mammography imaging, the radiation image may be interpreted while comparing a composite two-dimensional image obtained by tomosynthesis imaging with a two-dimensional image captured previously. However, due to a difference in a position of a radiation source during imaging, a positional relationship of a lesion, the overlapping of mammary glands, and the like may change, and it may be difficult to interpret the image.

The present disclosure has been made in consideration of the above-described circumstances, and an object of the present disclosure is to provide an image processing apparatus, an image processing method, and an image processing program that facilitate interpretation by comparing a composite two-dimensional image obtained by tomosynthesis imaging with a two-dimensional image captured previously.

In order to achieve the above-described object, a first aspect of the present disclosure provides an image processing apparatus comprising: a processor, in which the processor is configured to: acquire a two-dimensional image captured by irradiating a breast with radiation; acquire a position of a radiation source that emits the radiation in a case in which the two-dimensional image is captured; acquire a series of a plurality of projection images or a plurality of tomographic images obtained by performing tomosynthesis imaging of the breast; calculate a virtual projection position, which is a position at which the breast is virtually projected during the tomosynthesis imaging, from the position of the radiation source in a case in which the two-dimensional image is captured; and generate a composite two-dimensional image from the plurality of projection images or the plurality of tomographic images based on the virtual projection position.

A second aspect of the present disclosure provides the image processing apparatus according to the first aspect, in which the processor is configured to, in a case in which the plurality of projection images are acquired, generate the plurality of tomographic images from the plurality of projection images based on the virtual projection position and then generate the composite two-dimensional image from the plurality of tomographic images.

A third aspect of the present disclosure provides the image processing apparatus according to the second aspect, in which the processor is configured to generate the composite two-dimensional image from the plurality of tomographic images based on a projection path from the virtual projection position.

A fourth aspect of the present disclosure provides the image processing apparatus according to the second aspect, in which the processor is configured to: generate the plurality of tomographic images by correcting a magnification ratio using the virtual projection position as a center for each of the plurality of projection images; and generate the composite two-dimensional image by performing a parallel projection on the plurality of generated tomographic images.

A fifth aspect of the present disclosure provides the image processing apparatus according to the first aspect, in which the processor is configured to, in a case in which the plurality of tomographic images are acquired, generate the composite two-dimensional image by combining the plurality of tomographic images based on the virtual projection position.

An image processing apparatus according to a sixth aspect of the present disclosure provides the image processing apparatus according to the first aspect, in which the processor is configured to acquire the plurality of tomographic images of which magnification ratios are corrected using the virtual projection position as a center.

An image processing apparatus according to a seventh aspect of the present disclosure provides the image processing apparatus according to the first aspect, in which the processor is configured to: acquire the plurality of tomographic images and correct magnification ratios of the plurality of tomographic images at the virtual projection position in a case in which the magnification ratios of the plurality of tomographic images are not corrected or centers of the correction of the magnification ratios are at different positions; and generate the composite two-dimensional image by performing a parallel projection on the plurality of tomographic images subjected to the correction.

An image processing apparatus according to an eighth aspect of the present disclosure provides the image processing apparatus according to the first aspect, in which the processor is configured to: further acquire at least one of a position of the breast or an imaging angle of the breast in a case in which the two-dimensional image is captured, and calculate the virtual projection position from at least one of the position of the radiation source, the position of the breast, or the imaging angle of the breast in a case in which the two-dimensional image is captured.

An image processing apparatus according to a ninth aspect of the present disclosure provides the image processing apparatus according to the eighth aspect, in which the processor is configured to acquire at least one of the position of the radiation source, the position of the breast, or the imaging angle of the breast in a case in which the two-dimensional image is captured, from imaging information related to the two-dimensional image.

A tenth aspect of the present disclosure provides the image processing apparatus according to the eighth aspect, in which the processor is configured to acquire at least one of the position of the radiation source, the position of the breast, or the imaging angle of the breast in a case in which the two-dimensional image is captured, by calculating the position of the radiation source, the position of the breast, and the imaging angle of the breast from the two-dimensional image.

An eleventh aspect of the present disclosure provides the image processing apparatus according to the first aspect, in which the virtual projection position is a position that most matches the position of the radiation source in the two-dimensional image.

An image processing apparatus according to a twelfth aspect of the present disclosure provides the image processing apparatus according to the first aspect, in which the processor is configured to: acquire the two-dimensional image for each of a plurality of comparison targets; and generate the composite two-dimensional image corresponding to the two-dimensional image for each of the plurality of comparison targets.

A thirteenth aspect of the present disclosure provides the image processing apparatus according to the first aspect, in which the two-dimensional image includes at least one of a normal two-dimensional image captured by irradiating the breast with the radiation or the composite two-dimensional image obtained from the series of the plurality of projection images obtained by performing the tomosynthesis imaging of the breast.

A fourteenth aspect of the present disclosure provides an image processing method executed by a computer, the image processing method comprising: acquiring a two-dimensional image captured by irradiating a breast with radiation; acquiring a position of a radiation source that emits the radiation in a case in which the two-dimensional image is captured; acquiring a series of a plurality of projection images or a plurality of tomographic images obtained by performing tomosynthesis imaging of the breast; calculating a virtual projection position, which is a position at which the breast is virtually projected during the tomosynthesis imaging, from the position of the radiation source in a case in which the two-dimensional image is captured; and generating a composite two-dimensional image from the plurality of projection images or the plurality of tomographic images based on the virtual projection position.

A fifteenth aspect of the present disclosure provides an image processing program causing a computer to execute a process comprising: acquiring a two-dimensional image captured by irradiating a breast with radiation; acquiring a position of a radiation source that emits the radiation in a case in which the two-dimensional image is captured; acquiring a series of a plurality of projection images or a plurality of tomographic images obtained by performing tomosynthesis imaging of the breast; calculating a virtual projection position, which is a position at which the breast is virtually projected during the tomosynthesis imaging, from the position of the radiation source in a case in which the two-dimensional image is captured; and generating a composite two-dimensional image from the plurality of projection images or the plurality of tomographic images based on the virtual projection position.

According to the present disclosure, it is possible to facilitate interpretation by comparing the composite two-dimensional image obtained by tomosynthesis imaging with the two-dimensional image captured previously.

### BRIEF DESCRIPTION OF THE DRAWINGS

Fig. 1 is a configuration diagram schematically showing an example of an overall configuration of a radiographic imaging system according to an embodiment.
Fig. 2 is a diagram showing an example of tomosynthesis imaging.
Fig. 3 is a block diagram showing an example of a configuration of an image processing apparatus according to the embodiment.
Fig. 4 is a block diagram showing an example of a functional configuration of the image processing apparatus according to the embodiment.
Fig. 5A is a diagram showing examples of an irradiation position of a radiation source and a normal two-dimensional image.
Fig. 5B is a diagram showing virtual projection position calculation processing in a case in which the irradiation position of the radiation source is changed.
Fig. 5C is a diagram showing virtual projection position calculation processing in a case in which a position of a breast is changed.
Fig. 5D is a diagram showing virtual projection position calculation processing in a case in which an imaging angle of the breast is changed.
Fig. 6 is a diagram showing a magnification ratio.
Fig. 7 is a flowchart showing an example of a flow of processing by an image processing program according to the embodiment.
Fig. 8 is a flowchart showing another example of the flow of the processing by the image processing program according to the embodiment.

### DESCRIPTION OF THE PREFERRED EMBODIMENTS

Hereinafter, embodiments of the present disclosure will be described in detail with reference to the drawings. Each embodiment does not limit the present disclosure.

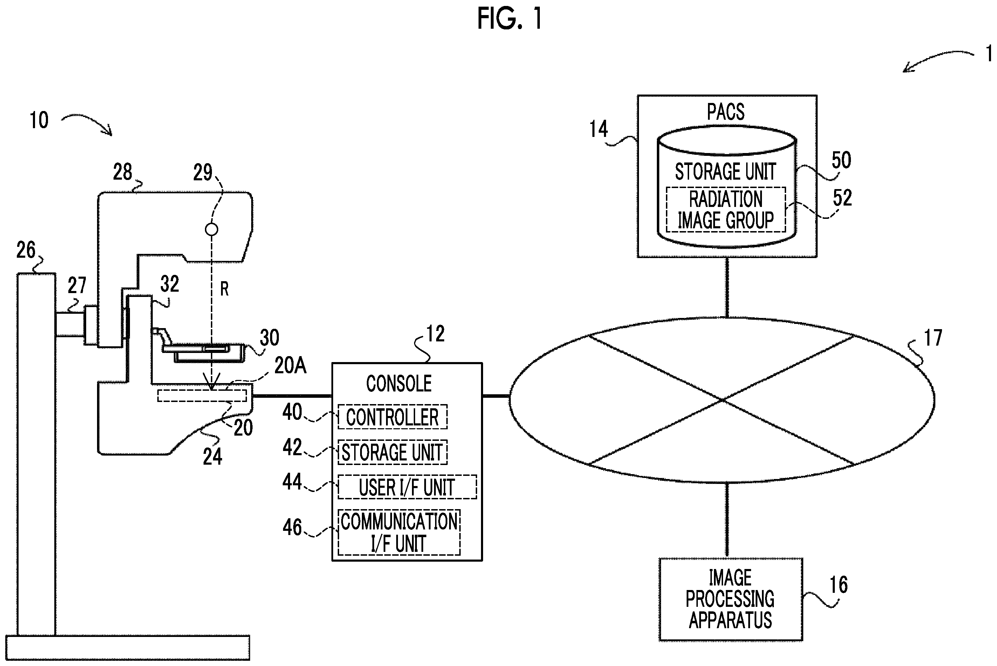

First, an example of an overall configuration of a radiographic imaging system according to the present embodiment will be described. Fig. 1 is a configuration diagram showing an example of an overall configuration of a radiographic imaging system 1 according to the present embodiment. As shown in Fig. 1, a radiographic imaging system 1 according to the present embodiment comprises a mammography apparatus 10, a console 12, a picture archiving and communication systems (PACS) 14, and an image processing apparatus 16. The console 12, the PACS 14, and the image processing apparatus 16 are connected via a network 17 by wired communication or wireless communication.

First, the mammography apparatus 10 according to the present embodiment will be described. In Fig. 1, a side view showing an example of an appearance of the mammography apparatus 10 according to the present embodiment is shown. It should be noted that Fig. 1 shows an example of the appearance in a case in which the mammography apparatus 10 is viewed from a left side of a person under examination.

The mammography apparatus 10 according to the present embodiment is an apparatus that is operated under the control of the console 12, and is configured to capture, using a breast of the person under examination as a subject, a radiation image of a breast by irradiating the breast with radiation R (for example, X-rays) emitted from a radiation source 29. It should be noted that the radiation source 29 is, for example, a tube that emits the radiation R. Further, the mammography apparatus 10 according to the present embodiment has a function of performing normal imaging for capturing images by arranging the radiation source 29 at an irradiation position along a normal direction to a detection surface 20A of a radiation detector 20 and so-called tomosynthesis imaging (which will be described below) for capturing images by moving the radiation source 29 to each of a plurality of irradiation positions.

As shown in Fig. 1, the mammography apparatus 10 comprises an imaging table 24, a base 26, an arm portion 28, and a compression unit 32.

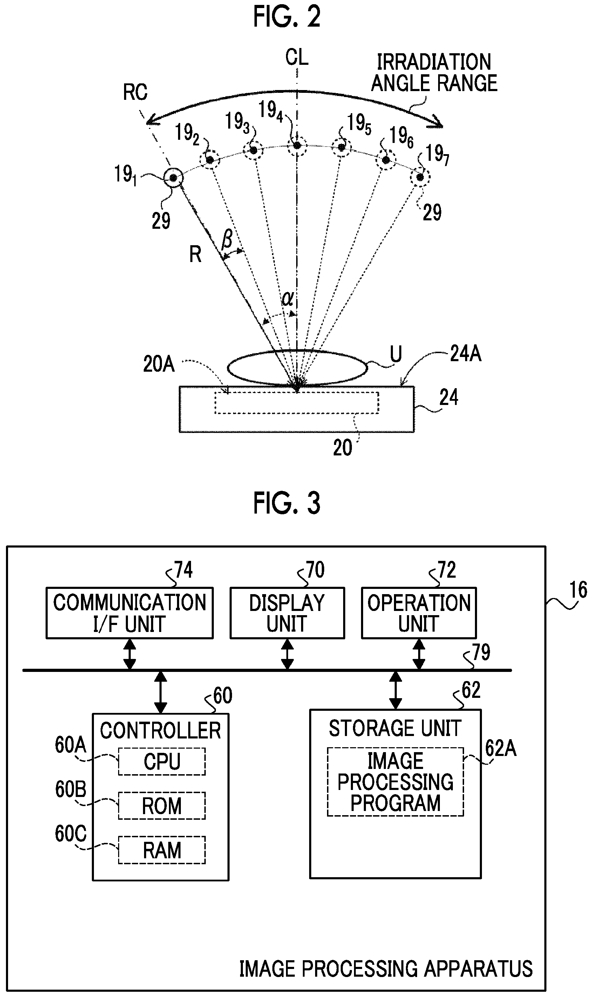

A radiation detector 20 is disposed inside the imaging table 24. As shown in Fig. 2, in the mammography apparatus 10 according to the present embodiment, in a case in which the imaging is performed, a breast U of the person under examination is positioned on an imaging surface 24A of the imaging table 24 by a user.

The radiation detector 20 detects the radiation R that has been transmitted through the breast U as the subject. Specifically, the radiation detector 20 detects the radiation R that enters the breast U of the person under examination and the imaging table 24 and that reaches the detection surface 20A of the radiation detector 20, generates a radiation image based on the detected radiation R, and outputs image data representing the generated radiation image. Hereinafter, the series of operations of irradiating the breast with the radiation R emitted from the radiation source 29 to generate the radiation image via the radiation detector 20 may be referred to as "imaging". A type of the radiation detector 20 according to the present embodiment is not particularly limited, and for example, the radiation detector 20 may be an indirect conversion type radiation detector that converts the radiation R into light and converts the converted light into charge, or may be a direct conversion type radiation detector that directly converts the radiation R into charge.

The compression plate 30 used to compress the breast in a case of performing imaging is attached to the compression unit 32 provided on the imaging table 24 and is moved in a direction approaching or departing from the imaging table 24 (hereinafter, referred to as an "up-down direction") by a compression plate drive unit (not shown) provided in the compression unit 32. The compression plate 30 is moved in the up-down direction to compress the breast of the person under examination between the imaging table 24 and the compression plate 30.

The arm portion 28 can rotate relative to the base 26 via a shaft portion 27. The shaft portion 27 is fixed to the base 26, and the shaft portion 27 and the arm portion 28 rotate as one body. Gears are provided in each of the shaft portion 27 and the compression unit 32 of the imaging table 24, and the gears are switched between an engaged state and a non-engaged state, so that a state in which the compression unit 32 of the imaging table 24 and the shaft portion 27 are connected to each other and are rotated integrally and a state in which the shaft portion 27 is separated from the imaging table 24 and idles can be switched. It should be noted that the elements for switching between transmission and non-transmission of power of the shaft portion 27 are not limited to the gears, and various mechanical elements can be used. The arm portion 28 and the imaging table 24 can be separately rotated relative to the base 26 with the shaft portion 27 as a rotation axis.

In a case in which the tomosynthesis imaging is performed in the mammography apparatus 10, the radiation source 29 is sequentially moved to each of the plurality of irradiation positions having different irradiation angles in accordance with the rotation of the arm portion 28. The radiation source 29 has a radiation tube (not shown) that generates the radiation R, and the radiation tube is moved to each of the plurality of irradiation positions in accordance with the movement of the radiation source 29. Fig. 2 is a diagram showing an example of the tomosynthesis imaging. It should be noted that, in Fig. 2, the compression plate 30 is not shown. In the present embodiment, as shown in Fig. 2, the radiation source 29 is moved to irradiation positions 19ₜ (t = 1, 2, ...; the maximum value is 7 in Fig. 2) having different irradiation angles at an interval of a predetermined angle β, that is, positions at which the irradiation angle of the radiation R with respect to the detection surface 20A of the radiation detector 20 are different from each other. At each irradiation position 19ₜ, the breast U is irradiated with the radiation R emitted from the radiation source 29 in accordance with an instruction of the console 12, and the radiation image is captured by the radiation detector 20. In the radiographic imaging system 1, in a case in which the tomosynthesis imaging is performed by moving the radiation source 29 to each irradiation position 19ₜ to capture the radiation image at each irradiation position 19ₜ, seven radiation images are obtained in the example of Fig. 2.

It should be noted that, during the tomosynthesis imaging, in a case in which the radiation image captured at each irradiation position 19 is described separately from other radiation images, the radiation image will be referred to as a "projection image", and a plurality of projection images captured in one tomosynthesis imaging will be referred to as a "series of a plurality of projection images".

It should be noted that, as shown in Fig. 2, the irradiation angle of the radiation R refers to an angle α formed between a normal line CL of the detection surface 20A of the radiation detector 20 and a radiation axis RC. The radiation axis RC refers to an axis that connects a focus of the radiation source 29 at each irradiation position 19 and a preset position, such as a center of the detection surface 20A. Further, here, it is assumed that the detection surface 20A of the radiation detector 20 is substantially parallel to the imaging surface 24A.

Meanwhile, in a case in which the mammography apparatus 10 performs the normal imaging, the radiation source 29 remains at the irradiation position 19ₜ (the irradiation position 19ₜ along the normal direction, the irradiation position 19₄ in Fig. 2) at which the irradiation angle α is 0 degrees. The radiation R is emitted from the radiation source 29 in accordance with the instruction of the console 12, and the radiation image is captured by the radiation detector 20. In the present embodiment, the radiation image captured during the normal imaging will be referred to as a "normal two-dimensional image" in a case in which the radiation image is described as being distinguished from other radiation images.

The mammography apparatus 10 and the console 12 are connected by wired communication or wireless communication. The radiation image captured by the radiation detector 20 in the mammography apparatus 10 is output to the console 12 by wired communication or wireless communication via a communication interface (I/F) unit (not shown).

As shown in Fig. 1, the console 12 according to the present embodiment comprises a controller 40, a storage unit 42, a user I/F unit 44, and a communication I/F unit 46.

As described above, the controller 40 of the console 12 has a function of controlling the capturing of the radiation image of the breast via the mammography apparatus 10. Examples of the controller 40 include a computer system comprising a central processing unit (CPU), a read only memory (ROM), and a random access memory (RAM).

The storage unit 42 has a function of storing, for example, information on the capturing of the radiation image, or the radiation image acquired from the mammography apparatus 10. The storage unit 42 is a non-volatile storage unit, and is, as an example, a hard disk drive (HDD) and a solid state drive (SSD).

The user I/F unit 44 includes input devices, such as various buttons and switches operated by the user such as a technician, regarding the capturing of the radiation image, and display devices, such as a lamp and a display, for displaying information on the imaging or the radiation image.

The communication I/F unit 46 performs communication of various types of data, such as information on the capturing of the radiation image or the radiation image obtained by the imaging, to and from the mammography apparatus 10 by wired communication or wireless communication. In addition, the communication I/F unit 46 performs communication of various types of data, such as the radiation image, with the PACS 14 and the image processing apparatus 16 via the network 17 by wired communication or wireless communication.

In addition, as shown in Fig. 1, the PACS 14 according to the present embodiment comprises a storage unit 50 that stores a radiation image group 52 and a communication I/F unit (not shown). The radiation image group 52 includes radiation images that are captured by the mammography apparatus 10 and that are acquired from the console 12 via a communication I/F unit (not shown) and the like.

The image processing apparatus 16 is used by a doctor or the like (hereinafter, simply referred to as "doctor") to interpret the radiation image. The image processing apparatus 16 according to the present embodiment has a function of generating a composite two-dimensional image from a series of a plurality of projection images or a plurality of tomographic images. The plurality of tomographic images can be obtained from the series of the plurality of projection images. The plurality of tomographic images are generated, for example, by reconstructing the series of the plurality of projection images using a simple back projection method, a filtered back projection method, a sequential reconstruction method, or the like. The "composite two-dimensional image" is a pseudo two-dimensional image generated by combining the plurality of tomographic images. The composite two-dimensional image is generated by combining the plurality of tomographic images having different distances (positions in a height direction) from the detection surface 20A of the radiation detector 20 to the radiation source 29 side, for example, by an addition method, an averaging method, a maximum value projection method, a minimum value projection method, or the like.

Fig. 3 is a block diagram showing an example of a configuration of the image processing apparatus 16 according to the present embodiment. As shown in Fig. 3, the image processing apparatus 16 according to the present embodiment comprises a controller 60, a storage unit 62, a display unit 70, an operation unit 72, and a communication I/F unit 74. The controller 60, the storage unit 62, the display unit 70, the operation unit 72, and the communication I/F unit 74 are connected to each other via a bus 79, such as a system bus or a control bus, such that various types of information can be transmitted and received.

The controller 60 controls the overall operation of the image processing apparatus 16. The controller 60 comprises a CPU 60A, a ROM 60B, and a RAM 60C. Various programs and the like used by the CPU 60A for the control are stored in the ROM 60B in advance. The RAM 60C temporarily stores various types of data.

The storage unit 62 is a non-volatile storage unit and is, as a specific example, an HDD or an SSD. The storage unit 62 stores an image processing program 62A according to the present embodiment.

The display unit 70 displays the radiation images or various types of information. The display unit 70 is not particularly limited, and various displays and the like may be used. In addition, the operation unit 72 is used by the doctor to input instructions for a diagnosis for a lesion of the breast using the radiation image, the user to input various types of information, or the like. The operation unit 72 is not particularly limited, and examples of the operation unit 72 include various switches, a touch panel, a touch pen, and a mouse. It should be noted that the display unit 70 and the operation unit 72 may be integrated into a touch panel display.

The communication I/F unit 74 performs communication of various types of information between the console 12 and the PACS 14 via the network 17 by wireless communication or wired communication.

As described above, in a case of interpreting the radiation image obtained by the mammography imaging, the interpretation may be performed while comparing the composite two-dimensional image obtained by the tomosynthesis imaging with the two-dimensional image captured previously. However, due to a difference in a position of a radiation source 29 during imaging, a positional relationship of a lesion, the overlapping of mammary glands, and the like may change, and it may be difficult to interpret the image.

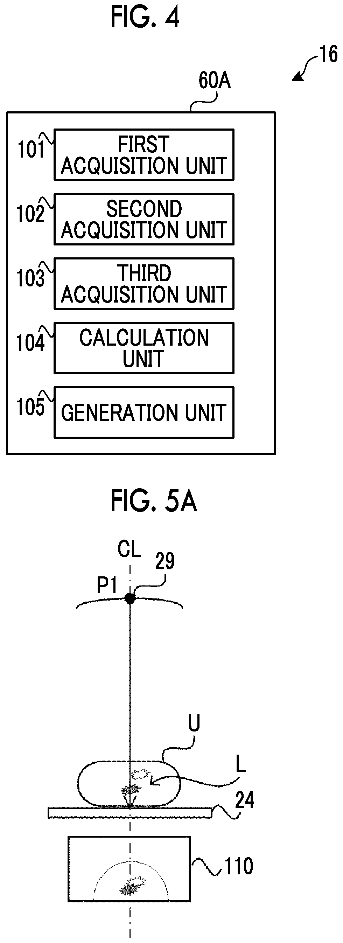

Therefore, the CPU 60A of the image processing apparatus 16 according to the present embodiment functions as each unit shown in Fig. 4 by writing the image processing program 62A stored in the storage unit 25 in the RAM 60C and executing the image processing program 62A.

Fig. 4 is a block diagram showing an example of a functional configuration of the image processing apparatus 16 according to the embodiment. The CPU 60A of the image processing apparatus 16 according to the present embodiment functions as a first acquisition unit 101, a second acquisition unit 102, a third acquisition unit 103, a calculation unit 104, and a generation unit 105. It should be noted that the first acquisition unit 101, the second acquisition unit 102, and the third acquisition unit 103 are distinguished for convenience, but may be implemented as one acquisition unit.

The first acquisition unit 101 acquires a two-dimensional image (hereinafter, referred to as a "previous two-dimensional image") captured by irradiating the breast U with the radiation R. The previous two-dimensional image usually includes at least one of the normal two-dimensional image or the composite two-dimensional image. The normal two-dimensional image is generally an image captured by irradiating the breast U with the radiation R. The composite two-dimensional image is an image obtained from the series of the plurality of projection images obtained by performing the tomosynthesis imaging of the breast U. The previous two-dimensional image is acquired from, for example, the PACS 14. In addition, in a case in which there are a plurality of comparison targets, the first acquisition unit 101 may acquire the previous two-dimensional image for each of the plurality of comparison targets.

The second acquisition unit 102 acquires the irradiation position of the radiation source 29 that emits the radiation R in a case in which the previous two-dimensional image is captured. Further, the second acquisition unit 102 may further acquire at least one of the position of the breast U or the imaging angle of the breast U in a case in which the previous two-dimensional image is captured. The second acquisition unit 102 may acquire at least one of the irradiation position of the radiation source 29, the position of the breast U, or the imaging angle of the breast U in a case in which the previous two-dimensional image is captured, from the imaging information related to the previous two-dimensional image, or by calculating the irradiation position of the radiation source 29, the position of the breast U, or the imaging angle of the breast U from the previous two-dimensional image. For example, the position of the breast U can be obtained from the center of the radiation detector 20 and a centroid position (for example, the nipple, the mammary gland, and the like) of the breast U.

The third acquisition unit 103 acquires the series of the plurality of projection images or the plurality of tomographic images. The plurality of projection images or the plurality of tomographic images are images obtained by performing the tomosynthesis imaging of the breast U.

The calculation unit 104 calculates a virtual projection position during the tomosynthesis imaging from the irradiation position of the radiation source 29 in a case in which the previous two-dimensional image is captured. Further, the calculation unit 104 may calculate the virtual projection position from at least one of the irradiation position of the radiation source 29, the position of the breast U, or the imaging angle of the breast U in a case in which the previous two-dimensional image is captured. The virtual projection position is a position at which the breast U is virtually projected during the tomosynthesis imaging. It is desirable that the virtual projection position is, for example, a position that most matches the irradiation position of the radiation source 29 previously two-dimensional image.

The generation unit 105 generates the composite two-dimensional image from the plurality of projection images or the plurality of tomographic images based on the virtual projection position calculated by the calculation unit 104. In addition, in a case in which the previous two-dimensional image is acquired for each of the plurality of comparison targets, the generation unit 105 may generate the composite two-dimensional image corresponding to the previous two-dimensional image for each of the plurality of comparison targets.

Here, virtual projection position calculation processing according to the present embodiment will be described in detail with reference to Figs. 5A to 5D.

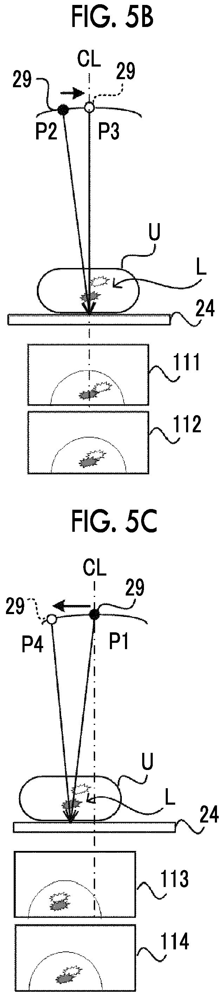

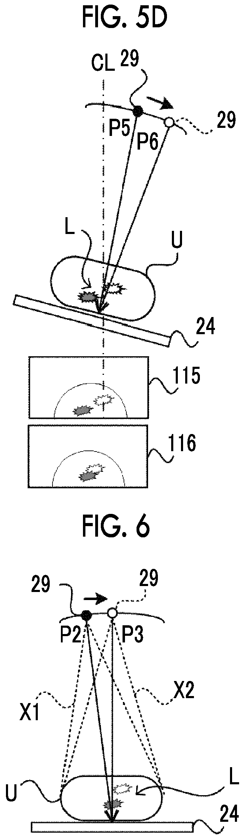

Fig. 5A is a diagram showing an example of an irradiation position P1 of the radiation source 29 and a normal two-dimensional image 110. Fig. 5B is a diagram showing the virtual projection position calculation processing in a case in which the irradiation position of the radiation source 29 is changed. Fig. 5C is a diagram showing the virtual projection position calculation processing in a case in which the position of the breast U is changed. Fig. 5D is a diagram showing the virtual projection position calculation processing in a case in which the imaging angle of the breast U is changed.

As shown in Fig. 5A, the irradiation position P1 of the radiation source 29 is, for example, a position on the normal line CL. The normal two-dimensional image 110 is generated by perform normal imaging of the breast U. The normal two-dimensional image 110 is an example of the previous two-dimensional image.

As shown in Fig. 5B, it is assumed that the irradiation position P1 of the normal two-dimensional image 110 is changed to an irradiation position P2 during the tomosynthesis imaging, that is, a tube position serving as a reference during the tomosynthesis imaging is shifted. In such a case, a projection image 111 is generated by imaging the breast U from the irradiation position P2 of the radiation source 29. In a case in which the projection image 111 and the normal two-dimensional image 110 are compared with each other, it can be seen that the positional relationship of the lesion L is changed. The calculation unit 104 calculates a corresponding virtual projection position P3 from the irradiation position P1 of the normal two-dimensional image 110 in order to match the positional relationship of the lesion L with the normal two-dimensional image 110. Specifically, for example, a difference between the irradiation position P1 of the normal two-dimensional image 110 and the irradiation position P2 of the projection image 111 may be obtained, and the irradiation position P2 may be corrected in accordance with the obtained difference. The virtual projection position P3 is calculated for each irradiation position during the tomosynthesis imaging based on the obtained difference. The virtual projection position P3 is a position at which the breast U is virtually projected during the tomosynthesis imaging. That is, since the tomosynthesis imaging is continuously performed at a plurality of irradiation positions, the virtual projection positions corresponding to the respective irradiation positions may be calculated by correcting the respective irradiation positions in accordance with the difference. The generation unit 105 generates a composite two-dimensional image 112 from the plurality of projection images or the plurality of tomographic images based on the calculated virtual projection position P3.

As shown in Fig. 5C, it is assumed that, during the tomosynthesis imaging, the position of the breast U in the normal two-dimensional image 110 is changed to the left side, that is, the position of the breast U is shifted. In such a case, a projection image 113 is generated by imaging the breast U from the irradiation position P1 of the radiation source 29. In a case in which the projection image 113 and the normal two-dimensional image 110 are compared with each other, it can be understood that the positional relationship of the lesion L is changed. The calculation unit 104 calculates a corresponding virtual projection position P4 from the position of the breast U of the normal two-dimensional image 110 in order to match the positional relationship of the lesion L with the normal two-dimensional image 110. Specifically, for example, a difference between the position of the breast U in the normal two-dimensional image 110 and the position of the breast U in the projection image 113 may be obtained, and the irradiation position P1 may be corrected in accordance with the obtained difference. The virtual projection position P4 is calculated for each irradiation position during the tomosynthesis imaging based on the obtained difference. The virtual projection position P4 is a position at which the breast U is virtually projected during the tomosynthesis imaging. The generation unit 105 generates a composite two-dimensional image 114 from the plurality of projection images or the plurality of tomographic images based on the calculated virtual projection position P4.

As shown in Fig. 5D, it is assumed that the imaging angle of the breast U of the normal two-dimensional image 110 is changed, that is, the imaging angle of the breast U is shifted. In such a case, a projection image 115 is generated by imaging the breast U from the irradiation position P5 of the radiation source 29. In a case in which the projection image 115 and the normal two-dimensional image 110 are compared with each other, it can be understood that the positional relationship of the lesion L is changed. The calculation unit 104 calculates a corresponding virtual projection position P6 from the imaging angle of the breast U of the normal two-dimensional image 110 in order to match the positional relationship of the lesion L with the normal two-dimensional image 110. Specifically, for example, a difference between the imaging angle of the breast U of the normal two-dimensional image 110 and the imaging angle of the breast U of the projection image 115 may be obtained, and the irradiation position P5 may be corrected in accordance with the obtained difference. The virtual projection position P6 is calculated for each irradiation position during the tomosynthesis imaging based on the obtained difference. The virtual projection position P6 is a position at which the breast U is virtually projected during the tomosynthesis imaging. The generation unit 105 generates a composite two-dimensional image 116 from the plurality of projection images or the plurality of tomographic images based on the calculated virtual projection position P6.

Next, a case will be described in which the projection image is acquired without acquiring the tomographic image and the composite two-dimensional image 112 is generated using the virtual projection position P3 as an example. The third acquisition unit 103 acquires the plurality of projection images. In a case in which the plurality of projection images are acquired, the generation unit 105 generates the plurality of tomographic images from the plurality of projection images based on the virtual projection position P3, and then generates the composite two-dimensional image 112 from the plurality of tomographic images.

Further, the generation unit 105 may generate the composite two-dimensional image 112 from the plurality of tomographic images based on a projection path from the virtual projection position P3. That is, the generation unit 105 generates the plurality of tomographic images from the plurality of projection images without correcting the magnification ratio, and projects the plurality of tomographic images from the virtual projection position P3 to generate the composite two-dimensional image 112.

Fig. 6 is a diagram showing the magnification ratio. The generation unit 105 may generate the plurality of tomographic images by correcting the magnification ratio using the virtual projection position P3 as a center for each of the plurality of projection images, and may generate the composite two-dimensional image 112 by performing a parallel projection on the plurality of generated tomographic images. In the example of Fig. 6, the irradiation position P2 of the radiation source 29 is corrected to the corresponding virtual projection position P3, but a magnification ratio X1 in a case in which the cone beam-shaped radiation is emitted using the irradiation position P2 as a center and a magnification ratio X2 in a case in which the cone beam-shaped radiation is emitted using the virtual projection position P3 as a center are different. Therefore, the plurality of tomographic images are generated by correcting the magnification ratio X1 to the magnification ratio X2 for each of the plurality of projection images.

Next, a case will be described in which the tomographic image is acquired instead of the projection image and the composite two-dimensional image 112 is generated using the virtual projection position P3 as an example. The third acquisition unit 103 acquires the plurality of tomographic images. In a case in which the plurality of tomographic images are acquired, the generation unit 105 generates a composite two-dimensional image 112 by combining the plurality of tomographic images based on the virtual projection position P3.

In addition, the third acquisition unit 103 may acquire the plurality of tomographic images of which the magnification ratios are corrected using the virtual projection position P3 as a center. In such a case, the generation unit 105 generates the composite two-dimensional image 112 by performing a parallel projection on the plurality of acquired tomographic images. That is, the magnification ratio may be corrected at the virtual projection position at which the tomographic image itself is calculated from the previous two-dimensional image, and the composite two-dimensional image may be generated from the tomographic images in which the magnification ratios are corrected.

In addition, in a case in which the magnification ratios of the plurality of acquired tomographic images are not corrected or the centers of the correction of the magnification ratios are at different positions, the generation unit 105 may correct the magnification ratios of the plurality of tomographic images at the virtual projection position P3 and perform a parallel projection on the plurality of corrected tomographic images to generate the composite two-dimensional image 112.

The image processing apparatus 16 according to the present embodiment may also be configured to acquire the previous two-dimensional image, the imaging information including the irradiation position of the radiation source 29, and the plurality of tomographic images from the PACS 14, for example. According to this configuration, for example, the composite two-dimensional image obtained by combining the plurality of acquired tomographic images at the virtual projection position can be generated on an image viewer, and the previous two-dimensional image and the composite two-dimensional image can be compared with each other and interpreted.

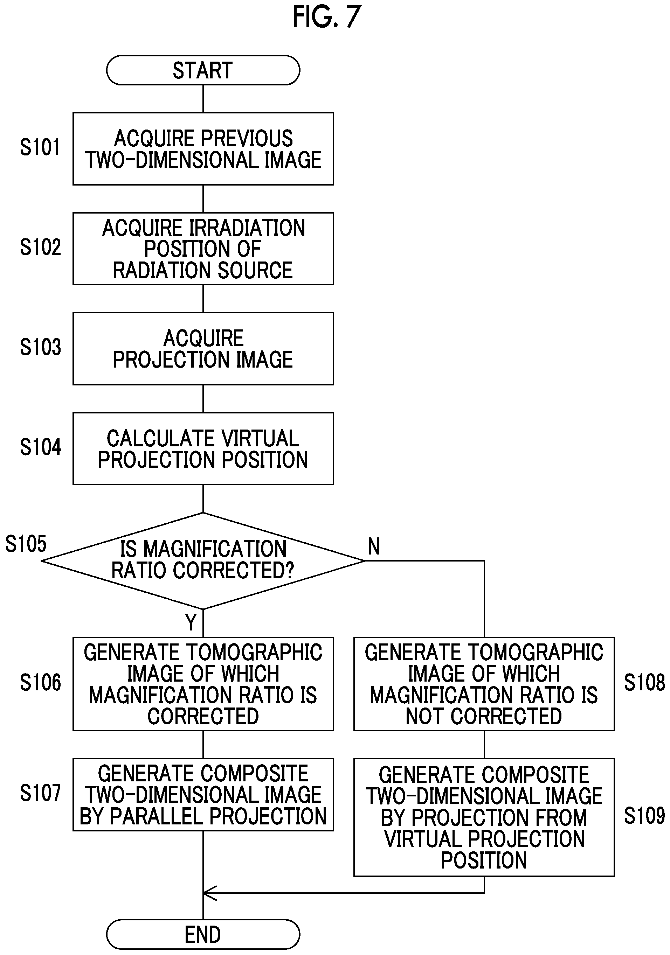

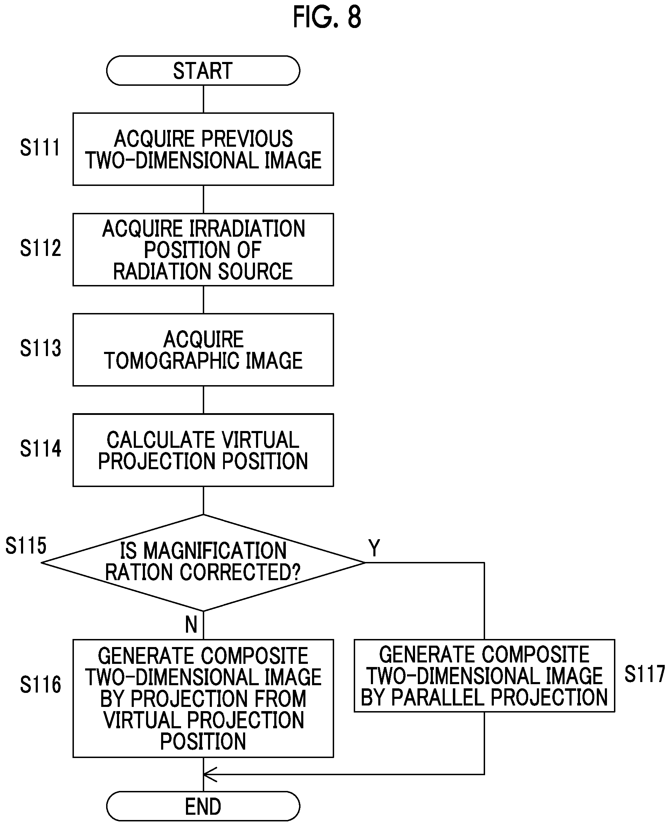

Next, an operation of the image processing apparatus 16 according to the present embodiment will be described with reference to Figs. 7 and 8.

Fig. 7 is a flowchart showing an example of a flow of the processing by the image processing program 62A according to the present embodiment. In Fig. 7, a case will be described in which the projection image is acquired without acquiring the tomographic image.

First, in a case in which the image processing apparatus 16 receives an instruction to start the image processing, the CPU 60A reads out the image processing program 62A and executes the image processing program 62A.

In step S101 of Fig. 7, the CPU 60A acquires the previous two-dimensional image from, for example, the PACS 14. As described above, the previous two-dimensional image may be, for example, the normal two-dimensional image or the composite two-dimensional image.

In step S102, the CPU 60A acquires the irradiation position of the radiation source 29 in a case in which the previous two-dimensional image is captured, based on, for example, the imaging information related to the previous two-dimensional image. It should be noted that the irradiation position of the radiation source 29 may be acquired by calculating from the previous two-dimensional image. Further, at least one of the irradiation position of the radiation source 29, the position of the breast U, or the imaging angle of the breast U may be acquired.

In step S103, the CPU 60A acquires the series of the plurality of projection images obtained by performing the tomosynthesis imaging of the breast U as a subject.

In step S104, the CPU 60A calculates the virtual projection position of the breast U during the tomosynthesis imaging from the irradiation position of the radiation source 29 in a case in which the previous two-dimensional image is captured. The virtual projection position is a position at which the breast U is virtually projected, and is, for example, a position that most matches the irradiation position of the radiation source 29 previously two-dimensional image.

In step S105, the CPU 60A determines whether or not to perform the correction of the magnification ratio using the virtual projection position as a center for each of the series of the plurality of projection images. In a case in which it is determined to perform the correction of the magnification ratio (in a case in which an affirmative determination is made), the processing proceeds to step S106, and in a case in which it is determined not to perform the correction of the magnification ratio (in a case in which a negative determination is made), the processing proceeds to step S108.

In step S106, the CPU 60A generates the plurality of tomographic images by correcting the magnification ratio using the virtual projection position as a center for each of the series of plurality of projection images.

In step S107, the CPU 60A generates the composite two-dimensional image by performing a parallel projection on the plurality of generated tomographic images, and completes the series of processing by the image processing program 62A.

On the other hand, in step S108, the CPU 60A generates the plurality of tomographic images for each of the series of plurality of projection images without correcting the magnification ratio.

In step S109, the CPU 60A generates the composite two-dimensional image by projecting the plurality of generated tomographic images from the virtual projection position, and completes the series of processing by the image processing program 62A.

Fig. 8 is a flowchart showing another example of the flow of the processing by the image processing program 62A according to the present embodiment. A case in which the tomographic image is acquired instead of the projection image will be described with reference to Fig. 8.

First, in a case in which the image processing apparatus 16 receives an instruction to start the image processing, the CPU 60A reads out the image processing program 62A and executes the image processing program 62A.

In step S111 of Fig. 8, the CPU 60A acquires the previous two-dimensional image from, for example, the PACS 14. As described above, the previous two-dimensional image may be, for example, the normal two-dimensional image or the composite two-dimensional image.

In step S112, the CPU 60A acquires the irradiation position of the radiation source 29 in a case in which the previous two-dimensional image is captured, based on, for example, the imaging information related to the previous two-dimensional image. It should be noted that the irradiation position of the radiation source 29 may be acquired by calculating from the previous two-dimensional image. Further, at least one of the irradiation position of the radiation source 29, the position of the breast U, or the imaging angle of the breast U may be acquired.

In step S113, the CPU 60A acquires the plurality of tomographic images from, for example, the PACS 14.

In step S114, the CPU 60A calculates the virtual projection position of the breast U during the tomosynthesis imaging from the irradiation position of the radiation source 29 in a case in which the previous two-dimensional image is captured. The virtual projection position is a position at which the breast U is virtually projected, and is, for example, a position that most matches the irradiation position of the radiation source 29 previously two-dimensional image.

In step S115, the CPU 60A determines whether or not the correction of the magnification ratio using the virtual projection position as a center is performed on the plurality of acquired tomographic images. In a case in which it is determined that the correction of the magnification ratio is not performed (in a case in which a negative determination is made), the processing proceeds to step S116, and in a case in which it is determined that the correction of the magnification ratio is performed (in a case in which an affirmative determination is made), the processing proceeds to step S117.

In step S116, the CPU 60A generates the composite two-dimensional image by projecting the plurality of acquired tomographic images from the calculated virtual projection position, and completes the series of processing by the image processing program 62A.

On the other hand, in step S117, the CPU 60A generates the composite two-dimensional image by performing a parallel projection on the plurality of acquired tomographic images, and completes the series of processing by the image processing program 62A.

It should be noted that, in a case in which the correction of the magnification ratio is not performed using the virtual projection position as a center for the plurality of tomographic images acquired in step S113 or the centers of the correction of the magnification ratios are at different positions, the magnification ratios of the plurality of tomographic images may be corrected by the virtual projection position calculated in step S114, and the composite two-dimensional image may be generated by the parallel projection.

As described above, according to the present embodiment, in a case in which the irradiation position of the radiation source during the tomosynthesis imaging is shifted with respect to the two-dimensional image captured previously, the virtual projection position at which the deviation of the irradiation position is corrected is calculated. Then, the composite two-dimensional image is generated from the series of the plurality of projection images or the plurality of tomographic images obtained by the tomosynthesis imaging, based on the calculated virtual projection position. The irradiation positions of the generated composite two-dimensional image are corrected in accordance with the previous two-dimensional image, and thus it is easy to compare the composite two-dimensional image with the previous two-dimensional image and interpret the composite two-dimensional image.

Although one form of the image processing apparatus 16 has been described above using the embodiment, the disclosed form of the image processing apparatus 16 is merely an example, and the form of the image processing apparatus 16 is not limited to the range described in the embodiment. Various modifications and improvements can be added to the embodiment without departing from the gist of the present disclosure, and the technical scope of the present disclosure also includes the embodiment to which the modifications or improvements are added.

In the above-described embodiment, as an example, a form has been described in which the control processing of the image processing apparatus 16 is implemented by software processing. However, the control processing of the image processing apparatus 16 may be implemented by hardware. In such a case, the processing speed is increased as compared with a case in which the processing is implemented by software processing.

In the above-described embodiment, the processor refers to a processor in a broad sense, and examples of the processor include a general-purpose processor (for example, the CPU), and a dedicated processor (for example, a graphics processing unit (GPU), an application specific integrated circuit (ASIC), a field programmable gate array (FPGA), a programmable logic device, or the like).

In addition, the operation of the processor in the embodiment described above may be performed not only by one processor but also by cooperation of a plurality of processors existing at physically separated positions. In addition, the order of the operations of the processor is not limited to only the order described in each of the embodiments described above, and may be changed as appropriate.

In the above-described embodiment, an example has been described in which the image processing program 62A is stored in the storage unit 62. However, a storage destination of the image processing program 62A is not limited to the storage unit 62. The image processing program 62A according to the present disclosure can also be provided in a form stored in a computer-readable storage medium. In addition, a form of a computer program product including the image processing program 62A may be adopted. The present disclosure is also applicable to a program and a program product.

For example, the image processing program 62A may be provided in a form of being stored in an optical disk, such as a CD-ROM, a DVD-ROM, and a Blu-ray disc. In addition, the image processing program 62A may be provided in a form of being stored in a portable semiconductor memory, such as a universal serial bus (USB) memory and a memory card. These CD-ROM, DVD-ROM, Blu-ray disc, USB, and memory card are examples of a non-transitory storage medium.

In regard to the embodiment described above, the supplementary notes will be further disclosed as follows.

### Supplementary Note 1

An image processing apparatus comprising: a processor, in which the processor is configured to: acquire a two-dimensional image captured by irradiating a breast with radiation; acquire a position of a radiation source that emits the radiation in a case in which the two-dimensional image is captured; acquire a series of a plurality of projection images or a plurality of tomographic images obtained by performing tomosynthesis imaging of the breast; calculate a virtual projection position, which is a position at which the breast is virtually projected during the tomosynthesis imaging, from the position of the radiation source in a case in which the two-dimensional image is captured; and generate a composite two-dimensional image from the plurality of projection images or the plurality of tomographic images based on the virtual projection position.

### Supplementary Note 2

The image processing apparatus according to supplementary note 1, in which the processor is configured to, in a case in which the plurality of projection images are acquired, generate the plurality of tomographic images from the plurality of projection images based on the virtual projection position and then generate the composite two-dimensional image from the plurality of tomographic images.

### Supplementary Note 3

The image processing apparatus according to supplementary note 2, in which the processor is configured to generate the composite two-dimensional image from the plurality of tomographic images based on a projection path from the virtual projection position.

### Supplementary Note 4

The image processing apparatus according to supplementary note 2, in which the processor is configured to: generate the plurality of tomographic images by correcting a magnification ratio using the virtual projection position as a center for each of the plurality of projection images; and generate the composite two-dimensional image by performing a parallel projection on the plurality of generated tomographic images.

### Supplementary Note 5

The image processing apparatus according to supplementary note 1, in which the processor is configured to, in a case in which the plurality of tomographic images are acquired, generate the composite two-dimensional image by combining the plurality of tomographic images based on the virtual projection position.

### Supplementary Note 6

The image processing apparatus according to supplementary note 1, in which the processor is configured to acquire the plurality of tomographic images of which magnification ratios are corrected using the virtual projection position as a center.

### Supplementary Note 7

The image processing apparatus according to supplementary note 1, in which the processor is configured to: acquire the plurality of tomographic images and correct magnification ratios of the plurality of tomographic images at the virtual projection position in a case in which the magnification ratios of the plurality of tomographic images are not corrected or centers of the correction of the magnification ratios are at different positions; and generate the composite two-dimensional image by performing a parallel projection on the plurality of tomographic images subjected to the correction.

### Supplementary Note 8

The image processing apparatus according to any one of supplementary notes 1 to 7, in which the processor is configured to: further acquire at least one of a position of the breast or an imaging angle of the breast in a case in which the two-dimensional image is captured, and calculate the virtual projection position from at least one of the position of the radiation source, the position of the breast, or the imaging angle of the breast in a case in which the two-dimensional image is captured.

### Supplementary Note 9

The image processing apparatus according to supplementary note 8, in which the processor is configured to acquire at least one of the position of the radiation source, the position of the breast, or the imaging angle of the breast in a case in which the two-dimensional image is captured, from imaging information related to the two-dimensional image.

### Supplementary Note 10

The image processing apparatus according to supplementary note 8, in which the processor is configured to acquire at least one of the position of the radiation source, the position of the breast, or the imaging angle of the breast in a case in which the two-dimensional image is captured, by calculating the position of the radiation source, the position of the breast, and the imaging angle of the breast from the two-dimensional image.

### Supplementary Note 11

The image processing apparatus according to any one of supplementary notes 1 to 10, in which the virtual projection position is a position that most matches the position of the radiation source in the two-dimensional image.

### Supplementary Note 12

The image processing apparatus according to any one of supplementary notes 1 to 11, in which the processor is configured to: acquire the two-dimensional image for each of a plurality of comparison targets; and generate the composite two-dimensional image corresponding to the two-dimensional image for each of the plurality of comparison targets.

### Supplementary Note 13

The image processing apparatus according to any one of supplementary notes 1 to 12, in which the two-dimensional image includes at least one of a normal two-dimensional image captured by irradiating the breast with the radiation or the composite two-dimensional image obtained from the series of the plurality of projection images obtained by performing the tomosynthesis imaging of the breast.

### Supplementary Note 14

An image processing method executed by a computer, the image processing method comprising: acquiring a two-dimensional image captured by irradiating a breast with radiation; acquiring a position of a radiation source that emits the radiation in a case in which the two-dimensional image is captured; acquiring a series of a plurality of projection images or a plurality of tomographic images obtained by performing tomosynthesis imaging of the breast; calculating a virtual projection position, which is a position at which the breast is virtually projected during the tomosynthesis imaging, from the position of the radiation source in a case in which the two-dimensional image is captured; and generating a composite two-dimensional image from the plurality of projection images or the plurality of tomographic images based on the virtual projection position.

### Supplementary Note 15

An image processing program causing a computer to execute a process comprising: acquiring a two-dimensional image captured by irradiating a breast with radiation; acquiring a position of a radiation source that emits the radiation in a case in which the two-dimensional image is captured; acquiring a series of a plurality of projection images or a plurality of tomographic images obtained by performing tomosynthesis imaging of the breast; calculating a virtual projection position, which is a position at which the breast is virtually projected during the tomosynthesis imaging, from the position of the radiation source in a case in which the two-dimensional image is captured; and generating a composite two-dimensional image from the plurality of projection images or the plurality of tomographic images based on the virtual projection position.

## Claims

1. An image processing apparatus (16) comprising:
a first acquisition unit (101) being configured to acquire a two-dimensional image captured by irradiating a breast (U) with radiation;
a second acquisition unit (102) being configured to acquire a position of a radiation source that emits the radiation in a case in which the two-dimensional image is captured;
a third acquisition unit (103) being configured to acquire a series of a plurality of projection images or a plurality of tomographic images obtained by performing tomosynthesis imaging of the breast (U);
a calculation unit (104) being configured to calculate a virtual projection position, which is a position at which the breast (U) is virtually projected during the tomosynthesis imaging, from the position of the radiation source in a case in which the two-dimensional image is captured; and
a generation unit (105) being configured to generate a composite two-dimensional image from the plurality of projection images or the plurality of tomographic images based on the virtual projection position.

2. The image processing apparatus (16) according to claim 1,
wherein the generation unit (105) is configured to, in a case in which the plurality of projection images are acquired, generate the plurality of tomographic images from the plurality of projection images based on the virtual projection position and then generate the composite two-dimensional image from the plurality of tomographic images.

3. The image processing apparatus (16) according to claim 2,
wherein the generation unit (105) is configured to generate the composite two-dimensional image from the plurality of tomographic images based on a projection path from the virtual projection position.

4. The image processing apparatus (16) according to claim 2,
wherein the generation unit (105) is configured to:
generate the plurality of tomographic images by correcting a magnification ratio using the virtual projection position as a center for each of the plurality of projection images; and
generate the composite two-dimensional image by performing a parallel projection on the plurality of generated tomographic images.

5. The image processing apparatus (16) according to claim 1,
wherein the third acquisition unit (103) is configured to, in a case in which the plurality of tomographic images are acquired, generate the composite two-dimensional image by combining the plurality of tomographic images based on the virtual projection position.

6. The image processing apparatus (16) according to claim 1,
wherein the third acquisition unit (103) is configured to acquire the plurality of tomographic images of which magnification ratios are corrected using the virtual projection position as a center.

7. The image processing apparatus (16) according to claim 1,
wherein the generation unit (105) is configured to:
acquire the plurality of tomographic images and correct magnification ratios of the plurality of tomographic images at the virtual projection position in a case in which the magnification ratios of the plurality of tomographic images are not corrected or centers of the correction of the magnification ratios are at different positions; and
generate the composite two-dimensional image by performing a parallel projection on the plurality of tomographic images subjected to the correction.

8. The image processing apparatus (16) according to any one of claims 1 to 7,
wherein the second acquisition unit (102) is configured to
further acquire at least one of a position of the breast (U) or an imaging angle of the breast (U) in a case in which the two-dimensional image is captured, and
the calculation unit (104) is configured to calculate the virtual projection position from at least one of the position of the radiation source, the position of the breast (U), or the imaging angle of the breast (U) in a case in which the two-dimensional image is captured.

9. The image processing apparatus (16) according to claim 8,
wherein the second acquisition unit (102) is configured to acquire at least one of the position of the radiation source, the position of the breast (U), or the imaging angle of the breast (U) in a case in which the two-dimensional image is captured, from imaging information related to the two-dimensional image.

10. The image processing apparatus (16) according to claim 8,
wherein the second acquisition unit (102) is configured to acquire at least one of the position of the radiation source, the position of the breast (U), or the imaging angle of the breast (U) in a case in which the two-dimensional image is captured, by calculating the position of the radiation source, the position of the breast (U), and the imaging angle of the breast (U) from the two-dimensional image.

11. The image processing apparatus (16) according to any one of claims 1 to 10,
wherein the virtual projection position is a position that most matches the position of the radiation source in the two-dimensional image.

12. The image processing apparatus (16) according to any one of claims 1 to 11, wherein the first acquisition unit (101) is configured to
acquire the two-dimensional image for each of a plurality of comparison targets; and
the generation unit (105) is configured to generate the composite two-dimensional image corresponding to the two-dimensional image for each of the plurality of comparison targets.

13. The image processing apparatus (16) according to any one of claims 1 to 12,
wherein the two-dimensional image includes at least one of a normal two-dimensional image captured by irradiating the breast (U) with the radiation or the composite two-dimensional image obtained from the series of the plurality of projection images obtained by performing the tomosynthesis imaging of the breast (U).

14. An image processing method executed by a computer (16), the image processing method comprising:
acquiring (S101, S111) a two-dimensional image captured by irradiating a breast (U) with radiation;
acquiring (S102, S112) a position of a radiation source that emits the radiation in a case in which the two-dimensional image is captured;
acquiring (S103, S113) a series of a plurality of projection images or a plurality of tomographic images obtained by performing tomosynthesis imaging of the breast (U);
calculating (S104, S114) a virtual projection position, which is a position at which the breast (U) is virtually projected during the tomosynthesis imaging, from the position of the radiation source in a case in which the two-dimensional image is captured; and
generating (S105-S109, S115-S117) a composite two-dimensional image from the plurality of projection images or the plurality of tomographic images based on the virtual projection position.

15. An image processing program (62A) causing a computer (16) to execute a process comprising:
acquiring a two-dimensional image captured by irradiating a breast (U) with radiation;
acquiring a position of a radiation source that emits the radiation in a case in which the two-dimensional image is captured;
acquiring a series of a plurality of projection images or a plurality of tomographic images obtained by performing tomosynthesis imaging of the breast;
calculating a virtual projection position, which is a position at which the breast (U) is virtually projected during the tomosynthesis imaging, from the position of the radiation source in a case in which the two-dimensional image is captured; and
generating a composite two-dimensional image from the plurality of projection images or the plurality of tomographic images based on the virtual projection position.
